# EUROPEAN PATENT SPECIFICATION

(11) **EP 1 495 056 B1**
(45) Date of publication and mention of the grant of the patent: **02.03.2011**
(21) Application number: 03745158.0
(22) Date of filing: 20.03.2003
(51) Int. Cl.: C07K 16/24, A61K 39/395, C12P 21/08

(54) **METHODS OF ANALYZING ANTIBODY DISULFIDE ISOMERS**
METHODEN ZUR ANALYSE VON ANTIKÖRPERDISULFIDISOMERE
METHODES D'ANALYSE D'ISOMERES DE DISULFURE D'ANTICORPS

(30) Priority: 20.03.2002 US 366350 P
(43) Date of publication of application: 12.01.2005
(73) Proprietor: UCB Pharma, S.A., 1070 Brussels (BE)
(72) Inventor: MOZIER, Ned, M., St. Charles, MI 63304 (US); DUFIELD, Robert, L., O'Fallon, MI 63366 (US); MO, Jianming, Chesterfield, MI 63017 (US); BILD, Gary, S., Chesterfield, MO 63005 (US)
(74) Representative: UCB Intellectual Property
(86) International application number: PCT/US2003/008608
(87) International publication number: WO 2003/080674

(56) References cited:
- WO-A-98/25971
- WO-A-03/099226
- WO-A1-01/94585
- CHOY E H S ET AL: "Efficacy of a novel PEGylated humanized anti-TNF fragment (CDP870) in patients with rheumatoid arthritis: A phase II double-blinded, randomized, dose-escalating trial" RHEUMATOLOGY (OXFORD), vol. 41, no. 10, October 2002 (2002-10), pages 1133-1137, XP002333059 ISSN: 1462-0324
- CHAPMAN A P: "PEGYLATED ANTIBODIES AND ANTIBODY FRAGMENTS FOR IMPROVED THERAPY: A REVIEW" ADVANCED DRUG DELIVERY REVIEWS, AMSTERDAM, NL, vol. 54, no. 4, 17 June 2002 (2002-06-17), pages 531-545, XP001199533 ISSN: 0169-409X
- RUSSELL ET AL.: 'Proteolysis in mixed organic-aqueous solvent systems: applications for peptide mass mapping using mass spectrometry' ANAL. CHEM. vol. 73, no. 11, 01 June 2001, pages 2682 - 2685, XP002970581

## Description

### FIELD OF THE INVENTION

The present invention relates to recombinant protein disulfide isomers and analytical methods of detecting the disulfide isomers and oxidated methionyl forms of the antibodies. More specifically, it relates to disulfide isomers of an antibody having specificity for antigenic determinants of human tumour necrosis factor alpha (TNFα). The present invention also relates to analytical methods of detecting the TNFα antibody disulfide isomers and oxidated methionyl forms of the TNFα antibodies. The present invention also relates to compositions comprising the isomers and therapeutic uses of the antibody.

### BACKGROUND OF THE INVENTION

In an antibody molecule, there are two heavy chains and two light chains. Each heavy chain and each light chain has at its N-terminal end a variable domain. Each variable domain is composed of four framework regions (FRs) alternating with three complementarily determining regions (CDRs). The residues in the variable domains are conventionally numbered according to a system devised by Kabat *et al.* This system is set forth in Kabat et al., 1987, in Sequences of Proteins of Immunological Interest, US Department of Health and Human Services, NIH, USA (hereafter "Kabat *et al. (supra)").* This numbering system is used in the present specification except where otherwise indicated.

The Kabat residue designations do not always correspond directly with the linear numbering of the amino acid residues. The actual linear amino acid sequence may contain fewer or additional amino acids than in the strict Kabat numbering corresponding to a shortening of, or insertion into, a structural component, whether framework or CDR, of the basic variable domain structure. The correct Kabat numbering of residues may be determined for a given antibody by alignment of residues of homology in the sequence of the antibody with a "standard" Kabat numbered sequence.

The CDRs of the heavy chain variable domain are located at residues 31-35 (CDRH1), residues 50-65 (CDRH2) and residues 95-102 (CDRH3) according to the Kabat numbering.

The CDRs of the light chain variable domain are located at residues 24-34 (CDRL1), residues 50-56 (CDRL2) and residues 89-97 (CDRL3) according to the Kabat numbering.

Construction of CDR-grafted antibodies is described in European Patent Application EP-A-0239400, which discloses a process in which the CDRs of a mouse monoclonal antibody are grafted onto the framework regions of the variable domains of a human immunoglobulin by site directed mutagenesis using long oligonucleotides. The CDRs determine the antigen binding specificity of antibodies and are relatively short peptide sequences carried on the framework regions of the variable domains.

The earliest work on humanising monoclonal antibodies by CDR-grafting was carried out on monoclonal antibodies recognising synthetic antigens, such as NP.

However, examples in which a mouse monoclonal antibody recognising lysozyme and a rat monoclonal antibody recognising an antigen on human T-cells were humanised by CDR-grafting have been described by Verhoeyen et al. (Science, 239, 1534-1536, 1988) and Riechmann et al. (Nature, 332, 323-324, 1988), respectively.

Riechmann *et al.,* found that the transfer of the CDRs alone (as defined by Kabat (Kabat *et al. (supra)* and Wu et al., J. Exp. Med., 132, 211-250, 1970)) was not sufficient to provide satisfactory antigen binding activity in the CDR-grafted product. It was found that a number of framework residues have to be altered so that they correspond to those of the donor framework region. Proposed criteria for selecting which framework residues need to be altered are described in International Patent Application WO 90/07861.

A number of reviews discussing CDR-grafted antibodies have been published, including Vaughan et al. (Nature Biotechnology, 16, 535-539, 1998).

TNFα is a pro-inflammatory cytokine that is released by and interacts with cells of the immune system. Thus, TNFα is released by macrophages that have been activated by lipopolysaccharides (LPS) of gram negative bacteria. As such, TNFα appears to be an endogenous mediator of central importance involved in the development and pathogenesis of endotoxic shock associated with bacterial sepsis. TNFα has also been shown to be up-regulated in a number of human diseases, including chronic diseases such as rheumatoid arthritis, Crohn's disease, ulcerative colitis, and multiple sclerosis. Mice transgenic for human TNFα produce high levels of TNFα constitutively and develop a spontaneous, destructive polyarthritis resembling rheumatoid arthritis (Kaffer et al., EMBO J., 10, 4025 4031, 1991). TNFα is therefore referred to as a pro-inflammatory cytokine.

Monoclonal antibodies against TNFα have been described in the prior art. Meager et al., (Hybridoma, 6, 305-311, 1987) describe murine monoclonal antibodies against recombinant TNFα. Fendly et al., (Hybridoma, 6, 359-370, 1987) describe the use of murine monoclonal antibodies against recombinant TNFα in defining neutralising epitopes on TNF. Shimamoto et al., (Immunology Letters, 17, 311-318, 1988) describe the use of murine monoclonal antibodies against TNF7 and their use in preventing endotoxic shock in mice. Furthermore, in International Patent Application WO 92/11383, recombinant antibodies, including CDR-grafted antibodies, specific for TNFα are disclosed. Rankin et al., (British J. Rheumatology, 34, 334-342, 1995) describe the use of such CDR-grafted antibodies in the treatment of rheumatoid arthritis. US-A-5 919 452 discloses anti-TNF chimeric antibodies and their use in treating pathologies associated with the presence of 5 TNF.

Antibodies to TNFα have been proposed for the prophylaxis and treatment of endotoxic shock (Beutler et al., Science, 234, 470-474, 1985). Bodmer et al., (Critical Care Medicine, 21, S441-S446, 1993) and Wherry et al., (Critical Care Medicine, 21, S436S440, 1993) discuss the therapeutic potential of anti-TNFα antibodies in the treatment of septic shock. The use of anti-TNFα antibodies in the treatment of septic shock is also discussed by Kirschenbaum et al., (Critical Care Medicine, 26, 1625-1626, 1998). Collagen-induced arthritis can be treated effectively using an anti-TNFα monoclonal antibody (Williams et al. (PNAS-USA, 89, 9784-9788, 1992)).

Increased levels of TNFα are found in both the synovial fluid and peripheral blood of patients suffering from rheumatoid arthritis. When TNFα blocking agents are administered to patients suffering from rheumatoid arthritis, they reduce inflammation, improve symptoms, and retard joint damage (McKown et al. (Arthritis Rheum., 42, 12041208, 1999).

The use of anti-TNFα antibodies in the treatment of rheumatoid arthritis and Crohn'S disease is discussed in Feldman et al., (Transplantation Proceedings, 30, 41264127, 1998), Adorini et al., (Trends in Immunology Today, 18, 209-211, 1997) and in Feldman et al., (Advances in Immunology, 64, 283-350, 1997). The antibodies to TNFα used in such treatments are generally chimeric antibodies, such.as those described in US-A5919452.

Two TNFα blocking products are currently licensed for the treatment of rheumatoid arthritis. The first, called etanercept, is marketed by Immunex Corporation as Enbrel™. It is a recombinant fusion protein comprising two p75 soluble TNF-receptor domains linked to the Fc portion of a human immunoglobulin. The second, called infliximab, is marketed by Centocor Corporation as Remicade™. It is a chimeric antibody having murine anti-TNFα variable domains and human IgG I constant domains.

The prior art recombinant anti-TNFα antibody molecules generally have a reduced affinity for TNFα compared to the antibodies from which the variable regions or CDRs are derived, generally have to be produced in mammalian cells and are expensive to manufacture. Prior art anti-TNFα, antibodies are described in Stephens et al., (Immunology, 85, 668-674, 1995), GB-A-2 246 570 and GB- A-2 297 145.

WO 01/94585 describes antibody molecules having high affinity for TNFα and low immunogenicity in humans, which can be used repeatedly and produced easily and efficiently, to treat chronic inflammatory diseases.

A method for proteolysis in mixed organic-aqueous solvent systems has been described (Russell, WK et al., Anal. Chem. 73:2682-2685, 2001). However, proteolysis of antibody fragments such as Fabs presents unique problems. The difference between the method of the present invention and the method of Russell et al. is the order of addition of the solvents. In the present method, Fab was dissolved in acetonitrile directly and then digestion buffer and enzyme were added. The method of Russell et al. would mix organic solvent and buffer first, then protein and enzyme were added into the solution. However, the method of Russell et al. would not digest Fab efficiently due the compact folded Fab structure.

### BREIF DESCRIPTION OF THE DRAWINGS

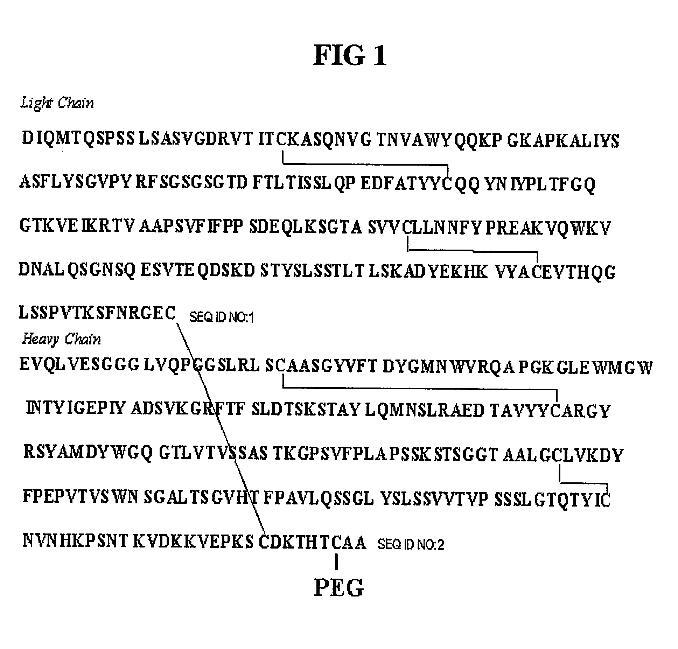

Figure 1 shows the amino acid sequence of the light and heavy chains of CDP870, the intramolecular disulfide pairing, the predominant intermolecular disulfide pairing between the light and heavy chains and the cysteine for PEG conjugation.

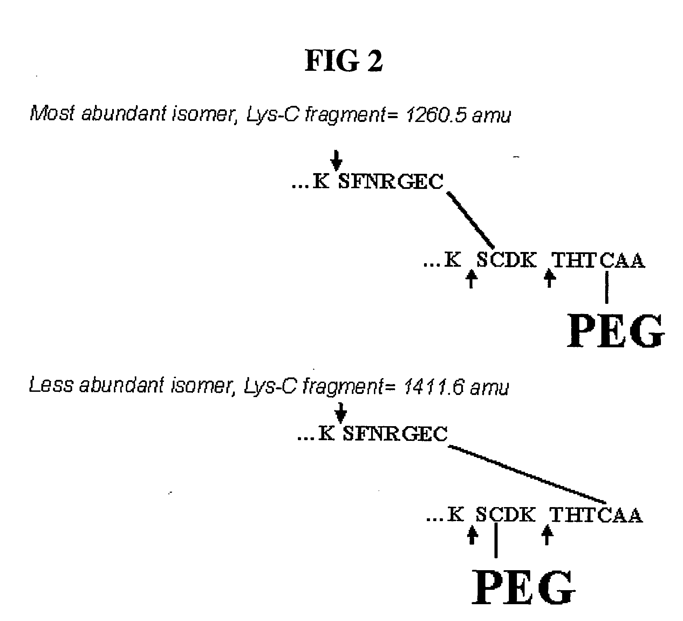

Figure 2 shows the designed disulfide pair Lys-C fragment of CDP870 and the isomer disulfide pair Lys-C fragment of CDP870, where the critical residues (light chain cys-214, heavy chain cys-221 and heavy chain cys-227 are highlighted. The arrows indicate the cleavage pattern of Lys-C.

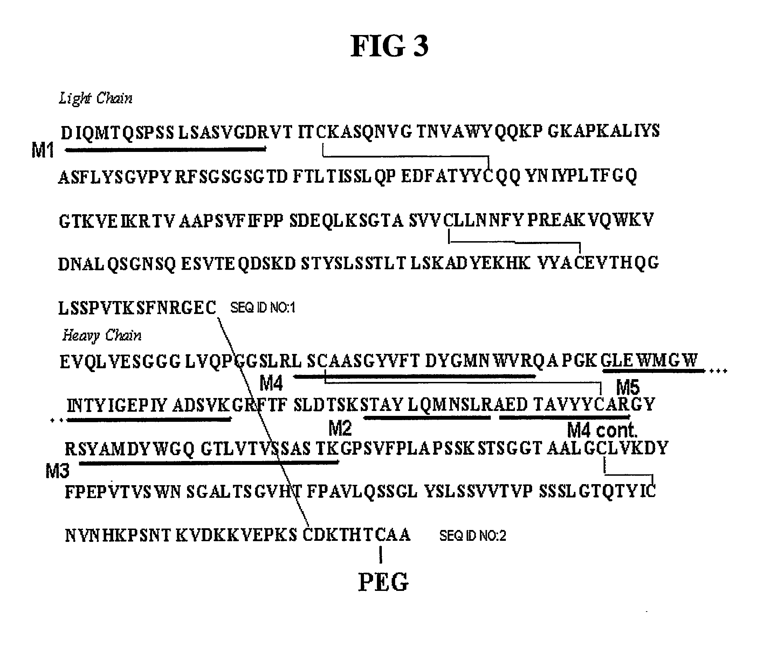

Figure 3 shows the methionine containing tryptic fragments of CDP870 in green.

Figure 4 shows the methionine Oxidation Tryptic Map for CDP870. Tryptic fragments where the methionyl residues are oxidized are described as O1-O5. For example, tryptic fragment M1, when oxidized, is described as O1, M2 as 02 and so on.

Figure 5 shows the disulfide isomer Lys-C Map for CDP870. The evidence for more abundance 1260.5 amu fragment relative to the 1411.6 amu fragment is apparent.

### SUMMARY OF THE INVENTION

The present invention relates to a method of detecting the antibody disulfide isomers comprising the steps of pre-treating the antibody with an organic solvent, digesting the antibody with a protease, and resolving the fragments. The present invention also relates to analytical methods of detecting the disulfide isomers and oxidated methionyl forms of the TNFα antibodies.

### DETAILED DESCRIBTION OF THE INVENTION

The present invention provides claim 1.

Another embodiment of the present invention is a method of analysis for the characterization and quantitation of disulfide isomers, methionyl oxidations, truncations, deamidation of asparagines, misincorporations, extensions, or other common protein degradations or protein impurities in recombinant proteins. Preferably, the protein is an antibody. More preferably, the antibody has specificity for TNFα. More preferably, the TNFα antibody is CDP870 as disclosed in WO 01/94585.

The present invention is a novel approach to digesting PEG-antibody fragment for analytical characterization, including but not limited to an Fab, modified Fab, Fab', F(ab')₂ or Fv fragment; a light chain or heavy chain monomer or dimer; a single chain antibody, and in this case CDP870. These compounds are very difficult to digest by conventional proteases due to the fact that PEG apparently hinders access of proteolytic enzymes to the protein backbone. This was solved by an assessment of the effect of organic solvents at precise quantities in the reaction mixture. It was discovered that acetonitrile and others were capable of modifying the 3-dimensional structure sufficient to allow complete digestion. Based on this finding, CDP870 could be digested by a number of proteases, including Lys C, and the cleaved protein analyzed by HPLC, mass spectrometry, and other means. This technique was applied to CDP870 and a disulfide isoform was found at approximately 10-20% comprised of material where the C-terminal cysteine residue of light chain was linked to cysteine residue 227 of heavy chain

The method of analysis comprises the steps of pre-treating the recombinant protein with an organic solvent, digesting the pre-treated protein with a protease in the presence of the organic solvent, and a means resolving the protease digest fragments. Preferably, the organic solvent is acetonitrile. Preferably the protease is trypsin for oxidation of methionyl residues and Lys-C for disulfide isomers.

Preferably, the means of resolving is reversed phase HPLC. The method of analysis was used to analyze and quantitate disulfide isomers and methionyl oxidations, but it could also be used for analysis of truncations, deamidation of asparagines, misincorporations, extensions, or other common protein degradations or protein impurities.

Although the foregoing invention has been described in some detail by way of illustration and example for the purposes of clarity of understanding, it will be readily apparent to one skilled in the art in light of the teachings of this invention that changes and modifications can be made without departing from the scope of the present invention. The following examples are provided for exemplification purposes only and are not intended to limit the scope of the invention, which has been described in broad terms above.

### EXAMPLES

### EXAMPLE 1

### Method for detecting disulfide isomer

A stock protein solution was prepared by diluting CDP870 to 20 mg/mL or CDP870 Fab' to 10 mg/mL in 50 mM acetate, 125 mM NaCl, pH 5.5 buffer. Lys-C was purchased from Wako (Osaka, Japan), prepared at 1 mg/mL in 100 mM Tris, pH 8.5.

### Pre-treatment with acetonitrile

20 µL of diluted sample was mixed with 40 µL acetonitrile, vortexed briefly and let stand for 5 minutes.

### Lys-C Digestion

130 µL of 100 mM Tris-HCl, pH 8.5 was added, mixed, then combined with 10 µL Lys-C stock solution. The Lys-C digestion mixture was incubated at 37°C for about 12 -16 hours.

### Reversed Phase HPLC

The digest is not quenched and 50 µL was injected directly on the HPLC column (Phenomenex Jupiter 300 angstroms, 5 µ, 2.1 mm x 250 mm) using a flow rate: 0.5 mL/minute. The Detection Wavelength was 214 nm. The Mobile Phase was A, 0.1 % TFA in water and B, 0.1 % TFA in acetonitrile. The Gradient (binary system) was as follows:

| Time | Mobile Phase B |
|---|---|
| 0 min. | 0% |
| 3 min | 6% |
| 8 min. | 10% |
| 9 min. | 11% |
| 21 min. | 15% |
| 21.1 min. | 95% |
| 27 min. | 95% |
| 27.1 min. | 0% |

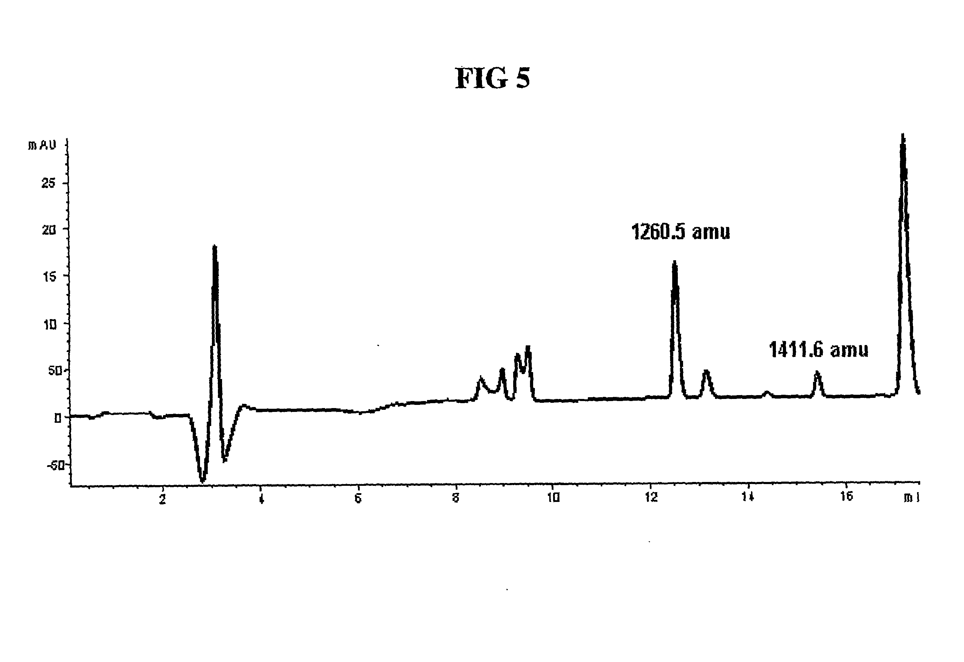

Figure 5 shows the disulfide isomer Lys-C Map for CDP870

### EXAMPLE 2

### Method for detecting methionine oxidation

A stock protein solution was prepared by diluting CDP870 to 20 mg/mL or CDP870 Fab' to 10 mg/mL in 50 mM acetate, 125 mM NaCl, pH 5.5 buffer. Trypsin solution was purchased from Promega (Madison WI) with a trypsin concentration of 0.4 mg/mL in 50 mM acetic acid buffer.

### Pre-treatment with acetonitrile

200 µL of diluted sample was mixed with 200 µL acetonitrile, vortexed briefly and let stand for 5 minutes.

### Trypsin Digestion

500 µL of digestion buffer (50 mM Tris-HCl, 1 mM CaCl₂, pH 8.5) was added, mixed and then combined with 100 µL trypsin stock solution. The trypsin digestion mixture was incubated at 37°C for about 12 -16 hours.

### Reversed Phase HPLC

The digest was quenched with 100 µL 1.0 N HCl and was injected directly on the HPLC column (Zorbax 300SB-C18 (Bodman; Aston, PA), 3.5 µ, 4.6 mm x 15 cm) using a flow rate: 0.5 mL/minute. The Detection Wavelength was 214 nm. The Mobile Phase was A, 0.1 % TFA in water and B, 0.1 % TFA in acetonitrile. The Gradient (binary system) was as follows:

| Time | Mobile Phase B | |
|---|---|---|
| 0 min. | 16% | |
| 15 min | 25% | Linear |
| 25 min. | 27% | Linear |
| 50 min. | 42% | Linear |
| 52 min. | 90% | Linear |
| 60 min. | 90% | Hold |
| 63 min. | 16% | Linear |
| 78 min. | 16% | Hold |

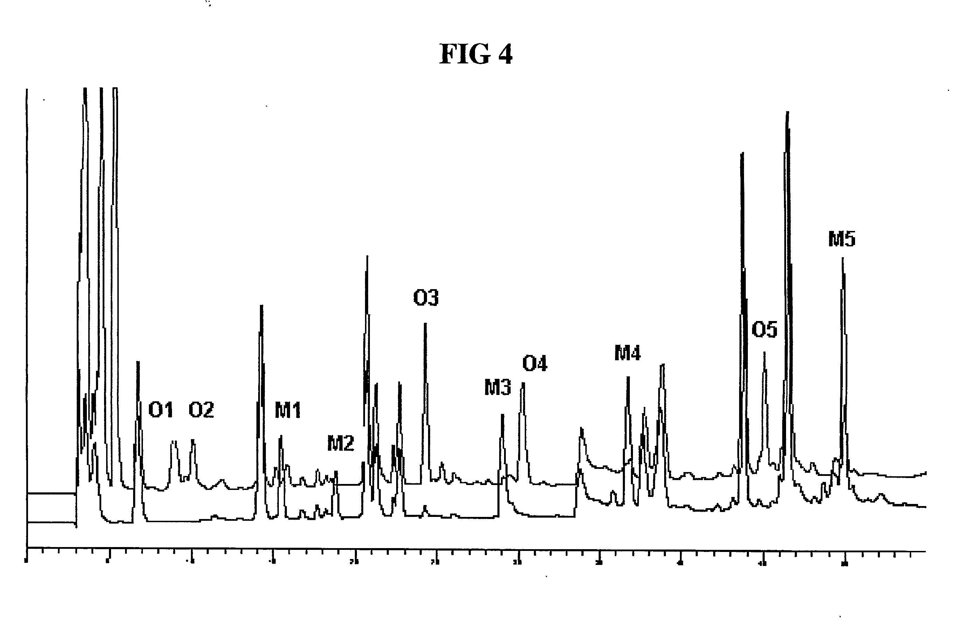

Figure 4 shows the methionine Oxidation Tryptic Map for CDP870

### EXAMPLE 3

### TNF Affinity Method

A TNFα affinity column was prepared by coupling human recombinant human TNFα (Biosource International, CA*) to UltraLink™ Biosupport Medium (Pierce, Rockford, IL) according to the instructions with the product. TNFα was dissolved in coupling buffer, 0.6 *M* sodium citrate, 50 *mM* CHES, pH 9.0. To make a 1 mL column, 0.126 g (dry weight) of UltraLink™ Biosupport was incubated with 750 µL of TNFα in coupling buffer. The reaction was incubated for 72 hours at room temperature with mixing. The beads were centrifuged using an Eppendorf 5415 microfuge at 1000 rpm, 4 °C for 5 minutes. The supernatant was decanted and the resin was resuspended in 10 mL of quench buffer to incubate for 2.5 hours at room temperature in a 15 mL screw top conical with gentle inversion as described previously. Quench buffer was 3 *M* ethanolamine, pH 9.0. After quenching the conical was centrifuged for 10 minutes (as above), and the supernatant decanted and the resin resuspended in 10 mL of phosphate buffered saline (PBS), pH 7.4 (Invitrogen™ life technologies, Carlsbad, CA) for 20 minutes at room temperature with gentle inversion. The washed beads were centrifuged for 10 minutes (as above), the supernatant decanted, and the beads re-suspended in 10 mL of 1 *M* sodium chloride and incubated for 20 minutes at room temperature with gentle mixing. The beads were centrifuged again, the supernatant decanted, and the beads re-suspended in 10 mL PBS, pH 7.4. This was mixed for 20 minutes, and this step repeated once more. The beads were then re-suspended in 4 mL of PBS, pH 7.4 and poured into a HR 5/5 column. The resulting 1 mL column was equilibrated in 50 column volumes (cv) of buffer A. Buffer A is 10 *mM* HEPES, 150 *mM* NaCl*,* pH 7.4.

The column was equilibrated, loaded, washed, eluted, and cleaned at 0.5 ml/min in line on an AKTA Explorer 100 Air with a UV900, pH, temperature, and conductivity meters and the Unicorn 4.0 operating system and analysis software. The column was equilibrated and washed in 10 *mM* HEPES, 150 *mM* NaCl, pH 7.4. The column was loaded with CDP870 (36426803, 200 mg/ml stock) that was diluted to 2 mg/ml with Buffer A and injected 0.5 ml/run. The column was eluted isocratically with 100 *mM* Glycine, pH 3.4. The column was cleaned with 10 mM HEPES, 2 *M* NaCl, pH 7.4. After loading, the flow-through (unbound) and also the eluted (bound) fractions were collected, and also the eluted fraction (bound). These two fractions and the column feed material were analyzed by LysC digestion followed by mass spectrometry. The peptide map profile showed similar amounts of peptides corresponding to Isomer #1 in all the samples, suggesting that Isomer#1 species were not different than parent in regards to binding TNFα.

## Claims

1. A method of analysis for the characterization and quantitation of antibody fragment disulfide isomers comprising the steps of: (a) pre-treating the antibody fragment with an organic solvent, (b) digesting the pre-treated antibody fragment with a protease in the presence of the organic solvent, and (c) resolving the protease digest fragments.

2. The method of claim 1 wherein said organic solvent is acetonitrile:

3. The method of claim 2 wherein said acetonitrile concentration in the pre-treatment step (a) is between about 40% and 80%.

4. The method of claim 3 wherein said acetonitrile concentration in the pre-treatment step (a) is about 67%.

5. The method of claim 2 wherein said acetonitrile concentration in the protease digestion step (b) is between about 20% and 50%.

6. The method of claim 3 wherein said acetonitrile concentration in the protease digestion step (b) is about 20%.

7. The method of claim 1 wherein said protease is Lys-C or trypsin.

8. The method of claim 1 wherein said protease digest fragments are resolved using reversed phase HPLC.

9. The method of any one of claims 1 to 8, wherein said antibody fragment is selected from the group consisting of: an Fab, modified Fab, Fab', F(ab')₂ or Fv fragment; a light chain or heavy chain monomer or dimer; and a single chain antibody.

10. The method of claim 9 wherein said antibody fragment is a Fab.

11. The method of claim 10 wherein said Fab is CDP870.

12. A method of analysis for the characterization and quantitation of antibody fragment degradation products and antibody fragment impurities in recombinant proteins selected from the group consisting of methionyl oxidations, truncations, deamidation of asparagines, misincorporations, extensions, or other common protein degradations or protein impurities comprising the steps of: (a) pre-treating the protein with an organic solvent, (b) digesting the pre-treated protein with a protease in the presence of the organic solvent, and (c) resolving the protease digest fragments.

13. The method of claim 12 wherein said acetonitrile concentration in the pre-treatment step (a) is about 50%.

14. The method of claim 13 wherein said acetonitrile concentration in the protease digestion step (b) is about 20%.

## Patentansprüche

1. Analyseverfahren zur Charakterisierung und Quantifizierung von Antikörperfragmentdisulfidisomeren, das die Schritte umfasst: (a) Vorbehandlung des Antikörperfragments mit einem organischen Lösungsmittel, (b) Verdau des vorbehandelten Antikörperfragments mit einer Protease in der Anwesenheit des organischen Lösungsmittels und (c) Auftrennen der Proteaseverdaufragmente.

2. Verfahren nach Anspruch 1, wobei das organische Lösungsmittel Acetonitril ist.

3. Verfahren nach Anspruch 2, wobei die Acetonitrilkonzentration im Vorbehandlungsschritt (a) zwischen etwa 40% und 80% ist.

4. Verfahren nach Anspruch 3, wobei die Acetonitrilkonzentration im Vorbehandlungsschritt (a) etwa 67% ist.

5. Verfahren nach Anspruch 2, wobei die Acetonitrilkonzentration im Proteaseverdauschritt (b) zwischen etwa 20% und 50% ist.

6. Verfahren nach Anspruch 3, wobei die Acetonitrilkonzentration im Proteaseverdauschritt (b) etwa 20% ist.

7. Verfahren nach Anspruch 1, wobei die Protease Lys-C oder Trypsin ist.

8. Verfahren nach Anspruch 1, wobei die Proteaseverdaufragmente mittels Umkehrphasen-HPLC aufgetrennt werden.

9. Verfahren nach einem der Ansprüche 1 bis 8, wobei das Antikörperfragment ausgewählt ist aus der Gruppe bestehend aus: einem Fab, modifiziertem Fab, Fab', F(ab')₂ oder Fv-Fragment; einem leichten oder schweren Kettenmonomer oder-dimer; und einem Einzelketten-Antikörper.

10. Verfahren nach Anspruch 9, wobei das Antikörperfragment ein Fab ist.

11. Verfahren nach Anspruch 9, wobei das Fab CDP870 ist.

12. Analyseverfahren zur Charakterisierung und Quantifizierung von Antikörperfragmentabbauprodukten und Antikörperfragmentverunreinigungen in rekombinanten Proteinen, ausgewählt aus der Gruppe bestehend aus Methionyloxidationen, Verkürzungen, Desamidierung von Asparaginen, Fehleinbauen, Verlängerungen oder anderen üblichen Proteinabbauprodukten oder Proteinverunreinigungen, das die Schritte umfasst: (a) Vorbehandlung des Proteins mit einem organischen Lösungsmittel, (b) Verdau des vorbehandelten Proteins mit einer Protease in der Anwesenheit des organischen Lösungsmittels und (c) Auftrennen der Proteaseverdaufragmente.

13. Verfahren nach Anspruch 12, wobei die Acetonitrilkonzentration im Vorbehandlungsschritt (a) etwa 50% ist.

14. Verfahren nach Anspruch 13, wobei die Acetonitrilkonzentration im Proteaseverdauschritt (b) etwa 20% ist.

## Revendications

1. Méthode d'analyse pour la caractérisation et quantification des isomères de disulfure de fragment d'anticorps comprenant les étapes suivantes :
(a) prétraiter le fragment d'anticorps avec un solvant organique,
(b) digérer le fragment d'anticorps prétraité avec une protéase en présence du solvant organique, et
(c) analyser les fragments digérés par la protéase.

2. Méthode selon la revendication 1, dans laquelle ledit solvant organique est l'acétonitrile.

3. Méthode selon la revendication 2, dans laquelle la concentration dudit acétonitrile dans l'étape de prétraitement (a) est entre environ 40% et 80%.

4. Méthode selon la revendication 3, dans laquelle la concentration dudit acétonitrile dans l'étape de prétraitement (a) est environ 67%.

5. Méthode selon la revendication 2, dans laquelle la concentration dudit acétonitrile dans l'étape de digestion par la protéase (b) est entre environ 20% et 50%.

6. Méthode selon la revendication 3, dans laquelle la concentration dudit acétonitrile dans l'étape de digestion par la protéase (b) est environ 20%.

7. Méthode selon la revendication 1, dans laquelle ladite protéase est Lys-C ou trypsine.

8. Méthode selon la revendication 1, dans laquelle lesdits fragments digérés par la protéase sont analysés en utilisant une HPLC en phase inverse.

9. Méthode selon l'une quelconque des revendications 1 à 8, dans laquelle ledit fragment d'anticorps est choisi dans le groupe constitué par : un fragment Fab, Fab modifié, Fab', F(ab')₂ ou Fv ; un monomère ou dimère de chaîne légère ou lourde ; et un anticorps à chaîne unique.

10. Méthode selon la revendication 9, dans laquelle ledit fragment d'anticorps est un Fab.

11. Méthode selon la revendication 10, dans laquelle ledit Fab est CDP870.

12. Méthode d'analyse pour la caractérisation et quantification des produits de dégradation de fragment d'anticorps et des impuretés de fragment d'anticorps dans les protéines recombinantes choisis dans le groupe constitué par des oxydations de methionyl, des troncatures, une désamidation d'asparagines, des misincorporations, des extensions, ou autres dégradations communes des protéines ou impuretés des protéines comprenant les étapes suivantes :
(a) prétraiter la protéine avec un solvant organique,
(b) digérer la protéine prétraitée avec une protéase en présence du solvant organique, et
(c) analyser les fragments digérés par la protéase.

13. Méthode selon la revendication 12, dans laquelle la concentration dudit acétonitrile dans l'étape de prétraitement (a) est environ 50%.

14. Méthode selon la revendication 13, dans laquelle la concentration dudit acétonitrile dans l'étape de digestion par la protéase (b) est environ 20%.


### SEQUENCE LISTING

<110> CELLTECH R&D LIMITED
<120> ANTIBODY DISULPHIDE ISOMERS, USE THEREOF, AND METHODS OF
   ANALYZING SAME
<130> P037674EP
<140> 03745158.0
   <141> 2003-03-20
<150> U.S 60/366350
   <151> 2002-03-20
<160> 2
<170> SeqWin99, version 1.02
<210> 1
   <211> 214
   <212> PRT
   <213> Artificial Sequence
<220>
   <223> CDP870 light chain
<400> 1
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Gln Asn Val Gly Thr Asn
               20                  25                  30
   Val Ala Trp Tyr Gln Gln Lys Pro Gly Lys Ala Pro Lys Ala Leu Ile
           35                  40                  45
   Tyr Ser Ala Ser Phe Leu Tyr Ser Gly Val Pro Tyr Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ile Tyr Pro Leu
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 2
   <211> 229
   <212> PRT
   <213> Artificial Sequence
<220>
   <223> CDP870 heavy chain
<400> 2
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Tyr Val Phe Thr Asp Tyr
               20                  25                  30
   Gly Met Asn Trp Val Arg Gln Ala Pro Gly Lys Gly Leu Glu Trp Met
           35                  40                  45
   Gly Trp Ile Asn Thr Tyr Ile Gly Glu Pro Ile Tyr Ala Asp Ser Val
       50                  55                  60
   Lys Gly Arg Phe Thr Phe Ser Leu Asp Thr Ser Lys Ser Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Gly Tyr Arg Ser Tyr Ala Met Asp Tyr Trp Gly Gln Gly Thr
               100                 105                 110
   Leu Val Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Ala Ala
   225